# FASCICULE DE BREVET EUROPEEN

(11) **EP 2 509 976 B1**
(45) Date de publication et mention de la délivrance du brevet: **25.03.2015**
(21) Numéro de dépôt: 10787766.4
(22) Date de dépôt: 07.12.2010
(51) Int. Cl.: C07D 401/14, C07D 249/06, C07F 15/00

(54) **COMPOSÉS À BASE D'UN GROUPEMENT TRIAZOLE APTES À COMPLEXER AU MOINS UN ÉLÉMENT METALLIQUE ET COMPLEXE DE COORDINATION À BASE DE CES COMPOSÉS**
VERBINDUNGEN AUF DER BASIS EINER TRIAZOLGRUPPE ZUR KOMPLEXIERUNG VON MINDESTENS EINEM METALLELEMENT UND KOORDINATIONSKOMPLEX AUF DER BASIS DIESER VERBINDUNGEN
COMPOUNDS BASED ON A TRIAZOLE GROUP THAT ARE CAPABLE OF COMPLEXING AT LEAST ONE METALLIC ELEMENT AND COORDINATION COMPLEX BASED ON THESE COMPOUNDS

(30) Priorité: 08.12.2009 FR 0958753
(43) Date de publication de la demande: 17.10.2012
(73) Titulaire: Commissariat à l'Énergie Atomique et aux Énergies Alternatives, 75015 Paris (FR); Centre National de la Recherche Scientifique, 75016 Paris (FR); UNIVERSITE PARIS-SUD (PARIS XI), 91405 Orsay Cédex (FR)
(72) Inventeur: LEIBL, Winfried, F-28210 Coulombs (FR); VAUZEILLES, Boris, F-92330 Sceaux (FR); BARON, Aurélie, F-95290 L'isle Adam (FR); AUKAULOO, Ally, F-91300 Massy (FR); HERRERO, Christian, F-75010 Paris (FR); CHARLOT, Marie-France, F-91400 Orsay (FR)
(74) Mandataire: Ilgart, Jean-Christophe
(86) Numéro de dépôt international: PCT/EP2010/069091
(87) Numéro de publication internationale: WO 2011/070025

(56) Documents cités:
- WINTER, ANDREAS ET AL: "Azido- and ethynyl-substituted 2,2':6',2''-terpyridines as suitable substrates for click reactions", SYNTHESIS , (9), 1506-1512 CODEN: SYNTBF; ISSN: 0039-7881, 25 mars 2009 (2009-03-25), XP002580328, cité dans la demande
- GLASNOV, TOMA N. ET AL: "Microwave-assisted Click chemistry for the preparation of 3- and 4-triazolyl-2(1H)-quinolones as potential fluorescent probes", QSAR & COMBINATORIAL SCIENCE , 26(11-12), 1261-1265 CODEN: QCSSAU; ISSN: 1611-020X, 5 septembre 2007 (2007-09-05), XP002580329,
- LACHAUD FABIEN ET AL: "A Biomimetic Model of the Electron Transfer between P680 and the TyrZ-His190 Pair of PSII", ANGEWANDTE CHEMIE. INTERNATIONAL EDITION, WILEY VCH VERLAG, WEINHEIM LNKD- DOI:10.1002/ANIE.200461948, vol. 44, 1 janvier 2005 (2005-01-01), pages 1536-1540, XP008090906, ISSN: 1433-7851 cité dans la demande
- DELAIN-BIOTON ET AL: "Synthesis of triazolyl-alkylphosphonate starting from omega-azidoalkylphosphonates or omega-alkynylphosphonates", TETRAHEDRON, ELSEVIER SCIENCE PUBLISHERS, AMSTERDAM, NL, vol. 63, no. 39, 16 August 2007 (2007-08-16), pages 9677-9684, XP022201057, ISSN: 0040-4020, DOI: 10.1016/J.TET.2007.07.024

## Description

### DOMAINE TECHNIQUE

La présente invention a trait à de nouveaux composés chimiques aptes à complexer au moins un élément métallique ainsi qu'à des complexes de coordination de ces composés avec un ou plusieurs éléments métalliques tels que le ruthénium.

Ces composés et les complexes de coordination à base de ceux-ci présentent potentiellement des propriétés photochimiques, c'est-à-dire qu'ils peuvent induire une conversion d'une énergie lumineuse en énergie chimique par la production, notamment, d'électrons et/ou de protons qui peuvent être impliqués ensuite dans des réactions chimiques, telles que des réactions chimiques d'oxydo-réduction.

De ce fait, ces composés peuvent trouver leur application dans de nombreux domaines impliquant la lumière comme source d'énergie, tels que le domaine de la production de carburants, le domaine de la production de produits de synthèse impliquant des réactions d'oxydo-réduction, le domaine thérapeutique ou du diagnostic impliquant également des réactions d'oxydo-réduction, le domaine de la production d'électricité.

### ÉTAT DE LA TECHNIQUE ANTÉRIEURE

De par leurs domaines d'applications très divers, de nombreuses équipes de recherche se sont ainsi fixé comme objectif de mettre au point des composés aptes, après fixation d'un élément métallique par complexation, à présenter des propriétés photochimiques.

C'est le cas notamment de Graetzel et al., (dans Coord.Chem.Rev., 2004, 248, 1447-1453), qui a mis en place un complexe à base de ruthénium de formule [Ru(Bipy(COO)₂)₂(NCS)₂], le ruthénium étant ainsi chélaté par deux groupes bipyridines porteurs de deux groupes carboxyles (symbolisé dans la formule par (Bipy(COO)₂)₂ et deux groupes isothiocyanato (symbolisé dans la formule par (NSC)₂)). Ce complexe est utilisé dans des dispositifs photovoltaïques, où il est adsorbé à la surface d'une électrode comprenant des grains de dioxyde de titane, cette électrode remplissant le rôle d'anode. Au contact d'une lumière visible, ce complexe absorbe des photons et éjecte, en retour, des électrons, qui sont captés par la bande de conduction du dioxyde de titane constitutif des grains de l'électrode, lesquels électrons sont ensuite véhiculés vers une cathode reliée à l'anode, où ils induisent la réduction d'un couple redox jouant le rôle d'un relais électronique (I₃⁻/I⁻).

Pour élargir le panel d'applications des composés aptes à complexer des éléments métalliques et pouvant présenter ainsi des propriétés photochimiques, certains auteurs ont travaillé sur la conception de composés organiques comprenant, outre des groupes complexants, d'autres groupes liés auxdits groupes complexants, notamment par le biais de groupes espaceurs, ces autres groupes pouvant présenter différentes fonctionnalités, telles que l'ancrage par liaison covalente sur divers supports, des propriétés catalytiques et/ou des propriétés d'adressage (c'est-à-dire une capacité à présenter une affinité pour une cible, telle qu'une cellule donnée, un compartiment cellulaire). Ces composés peuvent être qualifiés de composés modulaires.

Ainsi, certains auteurs ont développé de tels composés modulaires par association des groupes susmentionnés par le biais de groupes espaceurs, tels que des groupes espaceurs amidiques (comme décrit dans J.Am.Chem.Soc., 2000, 122, 3932-3936) ou des groupes espaceurs imidazoles (tels que décrits dans Angew. Chem. Int.Ed., 2005, 44, 1536-1540). Toutefois, la réalisation de tels composés en utilisant ce type de groupes espaceurs se fait dans des conditions difficiles à mettre en oeuvre, notamment, à grande échelle.

Pour palier ces inconvénients de synthèse, des auteurs ont mis en place des composés modulaires en couplant un groupe complexant avec un autre groupe en se basant sur une réaction de couplage entre lesdits groupes de mise en oeuvre simple et efficace : la réaction de cycloaddition 1,3-dipolaire de type Huisgen d'un groupe porteur d'une fonction azoture et d'un groupe porteur d'une fonction alcyne, moyennant quoi l'on obtient un composé comprenant un groupe complexant lié à un autre groupe par le biais d'un groupe espaceur triazole. C'est le cas notamment de Winter et al. dans Synthesis, 2009, n°9, p. 1506-1512, qui décrit des composés comprenant un groupe terpyridine lié à d'autres groupes (notamment des groupes aryles) par le biais d'un groupe espaceur triazole, le groupe terpyridine complexant du ruthénium. Les auteurs de cette publication ont fait le constat qu'il y avait une mauvaise communication électronique entre le groupe aryle et le groupe terpyridine séparés l'un de l'autre par un groupe triazole et ont attribué cette mauvaise conduction électronique aux mauvaises propriétés conductrices de ce groupe triazole.

Il existe donc, au vu de l'art antérieur, un véritable *a priori* technique à utiliser un groupe triazole formant pont entre deux groupes, dont l'un au moins des groupes est un groupe complexant d'au moins un élément métallique, pour obtenir des composés :
- présentant des propriétés photochimiques ; et
- présentant une bonne conduction électronique entre le groupe complexant et l'autre groupe via un groupe espaceur triazole.

Les auteurs de la présente invention se sont fixé pour objectif de proposer de nouveaux composés dont l'un au moins des groupes constitutifs dudit composé est un groupe complexant d'au moins un élément métallique, présentant après complexation avec un élément métallique, des propriétés photochimiques en présence d'un stimulus lumineux, lequel groupe complexant étant lié à un autre groupe pouvant présenter des fonctionnalités diverses (par exemple, une fonctionnalité d'ancrage sur un support, une fonctionnalité catalytique, etc) via un groupe espaceur, la conduction électronique devant être efficace entre le groupe complexant et ledit autre groupe.

### EXPOSÉ DE L'INVENTION

Les présents auteurs ont découvert, de manière surprenante et en dépit de l'*a priori* technique existant, qu'en choisissant un groupe complexant de façon judicieuse il est possible d'obtenir des composés comprenant ledit groupe complexant lié à un autre groupe via un groupe espaceur triazole, sans que la conduction électronique entre le groupe complexant et l'autre groupe soit interrompue ou amoindrie par le groupe espaceur triazole, comme cela est le cas avec les composés de l'art antérieur.

Ainsi, l'invention a trait à un composé répondant à la formule (I) suivante :

A-T-Z (I)

dans laquelle :
*A est un groupe apte à complexer au moins un élément métallique, qui est un groupe bipyridine;
*T est un groupe triazole directement lié au groupe A ;
*Z est un atome d'halogène, un groupe nitro, un groupe cyano, un groupe alkyle, un groupe aryle, un groupe hétérocyclique, un groupe de formule -NR¹R², un groupe de formule -SR¹, un groupe de formule -S(=O)₂(OR¹), un groupe de formule -O-S(=O)₂(OR¹), un groupe de formule -O-S(=O)₂(R¹), un groupe de formule -S(=O) (OR¹), un groupe de formule -S(=O) (R¹), un groupe de formule -S(=O)₂R¹, un groupe de formule -PR¹R², un groupe de formule -P(=O)(OR¹)(OR²), un groupe de formule -O-P(=O)(OR¹)(OR²), un groupe de formule -O-P(=O)(OR¹)(R²), un groupe de formule -OR¹ ou un groupe de formule -CO-R¹, R¹ et R² représentant indépendamment un atome d'hydrogène, un groupe alkyle ou un groupe aryle, lesdits groupes alkyles ou aryles étant éventuellement substitués.

En choisissant de façon appropriée le groupe A, les auteurs ont découvert, de façon surprenante, que ce groupe permettait d'améliorer la conductivité électronique du groupe triazole auquel il est lié, alors que ce groupe triazole est connu pour être un conducteur faible d'électrons, notamment lorsqu'il est lié à des groupes, tels qu'un groupe terpyridine. Les composés peuvent ainsi être utilisés comme des composés aptes à conduire des charges électroniques et à les véhiculer par exemple vers un dispositif sur lesquels ils peuvent être greffés (en choisissant de façon appropriée un groupe Z présentant des fonctions aptes à être greffées à la surface d'un tel dispositif).

Qui plus est, le groupe triazole est très stable thermiquement et chimiquement et résiste notamment à des conditions d'oxydation et de réduction très dures.

Le groupe A tel que mentionné ci-dessus présente également une capacité à complexer des éléments métalliques, tels que le ruthénium.

Les composés de l'invention sont également de conception simple, du fait de la possibilité de relier le groupe A au groupe Z par une simple réaction de couplage dite « réaction de click-chemistry » entre une fonction azoture et une fonction alcyne pour donner le groupe T triazole.

Selon l'invention, le groupe A des composés de l'invention est un groupe bipyridine ledit groupe pouvant être éventuellement substitué.

Par groupe bipyridine, on entend un groupe de formule suivante : 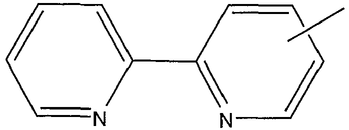 la liaison située au milieu de la liaison carbone-carbone indiquant que l'attachement au groupe triazole peut se faire par l'un quelconque des atomes de carbone constitutif du cycle bipyridine. Le groupe bipyridine susmentionné peut être substitué par un ou plusieurs substituants situé(s) au niveau des atomes de carbone constitutifs du cycle hormis celui qui est lié au groupe T.

Lorsque le groupe A est substitué, il peut être substitué par au moins un atome d'halogène et/ou au moins un groupe choisi parmi les groupes alkyle, aryle, amino, alcoxy ou hydroxy.

Avantageusement, le groupe A est un groupe bipyridine répondant à la formule suivante : 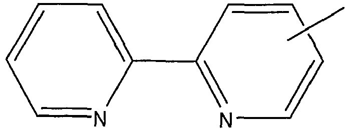 la liaison entrecoupant l'un des cycles indiquant que le groupe bipyridine peut être lié par l'un quelconque des atomes de carbone constitutif des cycles de la bipyridine au groupe T triazole.

Comme mentionné ci-dessus, le groupe T est un groupe triazole lié directement au groupe A susmentionné (c'est-à-dire qu'il est lié directement à l'un des atomes de carbone constitutif du ou des cycles aromatiques du groupe A), le groupe triazole répondant à la formule suivante : 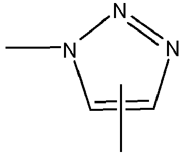 la liaison coupant la double liaison carbone-carbone indiquant que le groupe triazole est lié au groupe A ou groupe Z via l'un des atomes de carbone constitutif de cette double liaison tandis que la liaison attachée à l'atome d'azote indique que le triazole est lié à l'autre groupe (A ou Z) par le biais de cet atome d'azote.

Comme mentionné ci-dessus, Z peut être un atome d'halogène, un groupe nitro, un groupe cyano, un groupe alkyle, un groupe aryle, un groupe hétérocyclique, un groupe de formule -NR¹R², un groupe de formule -SR¹, un groupe de formule -S(=O)₂(OR¹), un groupe de formule -O-S(=O)₂(OR¹), un groupe de formule -O-S (=O)₂ (R¹), un groupe de formule -S(=O)(OR¹), un groupe de formule -S(=O)(R¹), un groupe de formule -S(=O)₂R¹, un groupe de formule -PR¹R², un groupe de formule -P(=O) (OR¹) (OR²), un groupe de formule -O-P(=O)(OR¹)(OR²), un groupe de formule -O-P (=O) (OR¹) (R²), un groupe de formule -OR¹ ou un groupe de formule -CO-R¹, R¹ et R² représentant indépendamment un atome d'hydrogène, un groupe alkyle ou un groupe aryle, lesdits groupes alkyles ou aryles étant éventuellement substitués (que ce soit pour le groupe Z lorqu'il représente un groupe alkyle ou aryle ou pour les groupes R¹ et R² lorsqu'ils représentent un groupe alkyle ou aryle).

Par atome d'halogène, on entend, classiquement, dans ce qui précède et ce qui suit, un atome pouvant être un atome de chlore, un atome de brome, un atome de fluor, un atome d'iode.

Par groupe nitro, on entend classiquement, dans ce qui précède et ce qui suit, un groupe de formule -NO₂.

Par groupe cyano, on entend classiquement, dans ce qui précède et ce qui suit, un groupe de formule -CN.

Par groupe alkyle éventuellement substitué, on précise qu'il peut s'agir d'un groupe alkyle linéaire ou ramifié, comprenant, par exemple de 1 à 6 atomes de carbone, tel qu'un groupe méthyle, éthyle, n-propyle, i-propyle, n-butyle, i-butyle, t-butyle, au moins un des atomes d'hydrogène du groupe alkyle pouvant être substitué, par exemple, par un atome d'halogène, un groupe nitro, un groupe cyano, un groupe alkyle, un groupe aryle, un groupe hétérocyclique, un groupe de formule -NR¹R², un groupe de formule -SR¹, un groupe de formule -S(=O)₂(OR¹), un groupe de formule -O-S (=O)₂ (OR¹) , un groupe de formule -O-S(=O)₂(R¹), un groupe de formule -S(=O)(OR¹), un groupe de formule -S(=O)(R¹), un groupe de formule -S(=O)₂R¹, un groupe de formule -PR¹R², un groupe de formule -P(=O)(OR¹)(OR²), un groupe de formule -O-P(=O)(OR¹)(OR²), un groupe de formule -O-P(=O)(OR¹)(R²), un groupe de formule -OR¹ ou un groupe de formule -CO-R¹, un groupe de formule -CO₂R¹, R¹ et R² représentant indépendamment un atome d'hydrogène, un groupe alkyle ou un groupe aryle, lesdits groupes alkyles ou aryles étant éventuellement substitués.

Par groupe aryle éventuellement substitué, on précise qu'il peut s'agir d'un groupe aryle comprenant de 6 à 18 atomes de carbone, tel qu'un groupe phényle, un groupe naphtyle, au moins un des atomes d'hydrogène portés par au moins un des cycles aromatiques du groupe étant éventuellement substitué, par exemple, par un atome d'halogène, un groupe nitro, un groupe cyano, un groupe alkyle, un groupe aryle, un groupe hétérocyclique, un groupe de formule -NR¹R², un groupe de formule -SR¹, un groupe de formule -S(=O)₂(OR¹), un groupe de formule -O-S(=O)₂(OR¹), un groupe de formule -O-S(=O)₂(R¹), un groupe de formule -S(=O)(OR¹), un groupe de formule -S(=O)(R¹), un groupe de formule -S(=O)₂R¹, un groupe de formule -PR¹R², un groupe de formule -P(=O)(OR¹)(OR²), un groupe de formule -O-P (=O) (OR¹) (OR²), un groupe de formule -O-P (=O) (OR¹) (R²), un groupe de formule -OR¹ ou un groupe de formule -CO-R¹, un groupe de formule -CO₂R¹, R¹ et R² représentant indépendamment un atome d'hydrogène, un groupe alkyle ou un groupe aryle, lesdits groupes alkyles ou aryles étant éventuellement substitués.

Avantageusement, Z peut être un groupe aryle, éventuellement substitué par au moins un atome d'halogène et/ou au moins un groupe -NR¹R² ou -CO₂R¹, R¹ et R² étant tels que définis ci-dessus.

De par sa nature chimique le groupe Z peut remplir différentes fonctions :
- Z peut être destiné à permettre le greffage des composés de l'invention sur un support, ce greffage intervenant par réaction chimique entre une fonction portée par le groupe Z et une fonction du support, le support pouvant être, par exemple, un support en silice, un support en oxyde de titane, un support en silicium, un support métallique (tel qu'un support en or), un support en carbone, un support en oxyde d'étain et d'indium (intitulé également support ITO pour « Indium Tin Oxide »), lequel support peut être une électrode d'un dispositif photovoltaïque ;
- Z peut être destiné à remplir une fonction catalytique, à savoir que le groupe Z peut amener des électrons provenant du groupe A, après complexation de ce dernier à un élément métallique suivie d'une excitation lumineuse, vers d'autres composés pour assurer une réaction d'oxydo-réduction de ces composés ;
- Z peut être destiné à remplir une fonction de contrôle des propriétés ou de l'activité d'un autre groupe auquel il est lié, par exemple du groupe A ;
- Z peut être destiné à remplir une fonction d'adressage, à savoir qu'il peut présenter une affinité chimique pour une cible donnée, telle qu'une cellule donnée, un compartiment cellulaire donné, une molécule donnée (telle qu'une molécule d'acide nucléique).

Des composés conformes à l'invention et particulièrement avantageux sont des composés pour lesquels A est un groupe bipyridine et Z est un groupe aryle éventuellement substitué.

Des composés répondant à cette définition sont des composés répondant à la formule (II) suivante :

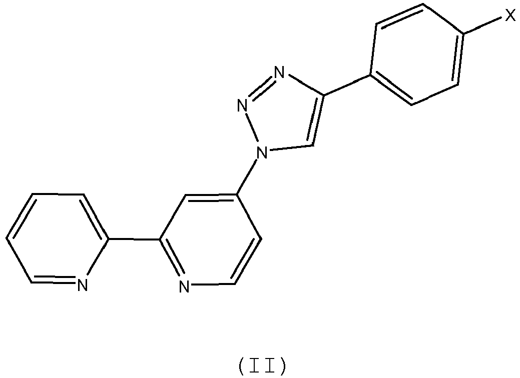

X représentant un atome d'hydrogène, un atome d'halogène, un groupe -NR¹R² ou un groupe -CO₂R¹, R¹ et R² répondant à la même définition que celle donnée ci-dessus.

Tout particulièrement, X peut être un atome d'hydrogène, un atome de fluor, un groupe -N(CH₃)₂ ou un groupe -CO₂-CH₃.

Comme mentionné ci-dessus, les composés de l'invention sont de conception aisée, dans la mesure où ils peuvent être synthétisés par une simple réaction de couplage entre une fonction azoture (ou alcyne) portée par un groupe A ou précurseur de celui-ci et une fonction alcyne (ou azoture) portée par un groupe Z ou précurseur de celui-ci selon le principe de la cycloaddition 1,3-dipolaire de type Huisgen, moyennant quoi l'on obtient le groupe triazole T formant pont entre le groupe A et le groupe Z. Cette réaction de couplage peut être catalysée par du cuivre sous forme de sel (tel que du sulfate de cuivre en présence d'un réducteur tel que l'ascorbate de sodium, ou encore tel que du iodure de cuivre CuI ou du bromure de cuivre CuBr). Cette réaction peut être mise en oeuvre dans un solvant comprenant de l'eau.

Comme mentionné ci-dessus, les composés de l'invention comprennent un groupe A spécifique apte à complexer au moins un élément métallique, c'est-à-dire à lier au moins un élément métallique par au moins une liaison de coordination.

Ainsi, l'invention a trait, selon un second objet, à des complexes de coordination d'au moins un élément métallique avec au moins un composé de l'invention tel que défini ci-dessus.

On précise que, par complexe de coordination, on entend classiquement un édifice polyatomique comprenant l'élément métallique autour duquel des groupes appartenant aux composés de l'invention (en l'occurrence, dans notre cas, le groupe A) sont liés par des liaisons de coordination, la liaison de coordination étant créée par apport d'un doublet d'électrons appartenant auxdits groupes dans une orbitale vide de l'élément métallique.

L'élément métallique peut un métal de transition, tel que Ti, Zr, Hf, V, Nb, Re, Ru, Ta, un élément lanthanide, un élément actinide ainsi que les éléments Al, Ga, Ge, In, Sn, Sb, Tl, Pb, Bi et Po.

En particulier, l'élément métallique est avantageusement un métal de transition, tel que le ruthénium Ru.

Outre les composés conformes à l'invention, les complexes définis ci-dessus peuvent comprendre, d'autres composés que ceux de l'invention aptes à complexer ledit élément métallique, tels que des composés ligands comme la pyridine et la bipyridine.

Des composés de l'invention particulièrement avantageux aptes à entrer dans la constitution de complexes de l'invention sont des composés de formule (II) suivante :

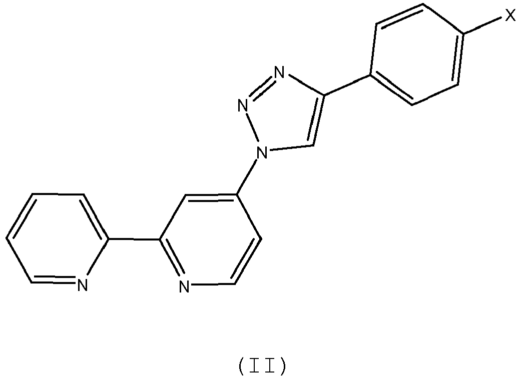

X représentant un atome d'hydrogène, un atome d' halogène, un groupe -NR¹R² ou un groupe -CO₂R¹, R¹ et R² répondant à la même définition que celle donnée ci-dessus, ces composés pouvant être utilisés en association dans le même complexe avec d'autres composés tels que la bipyrine.

Ainsi, des complexes avantageux conformes à la présente invention peuvent répondre à la formule (III) suivante : 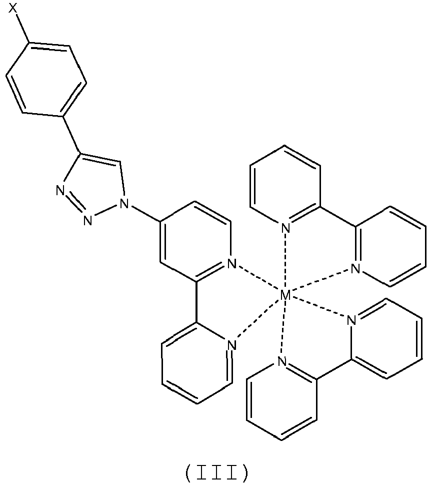 avec X étant tel que défini ci-dessus et M étant un élément métallique tel que le ruthénium, les liaisons en pointillés représentant des liaisons de coordination.

En particulier, X peut être un atome d'hydrogène, un atome de fluor, un groupe diéthylamino -N(CH₃)₂ ou un groupe -CO₂CH₃.

Avec de tels complexes, l'élément métallique, sous l'action de photons, peut ainsi se trouver sous un état excité libérant ainsi un électron qui peut circuler via le groupe triazole, bon conducteur d'électrons grâce au choix spécifique du groupe A, et via le groupe Z, le transfert se faisant de façon rapide notamment lorsque le groupe Z comporte une fonction acceptrice d'électrons (tel qu'un atome d'halogène comme le fluor).

Lorsque le groupe Z comporte une fonction donneuse d'électrons (notamment lorsque Z est un groupe phényle substitué par au moins un groupe -NR¹R²), il peut se produire, sous l'action de photons, un transfert rapide d'électrons du groupe Z vers le groupe A complexé avec un élément métallique.

L'on peut en déduire ainsi que le groupe triazole formant un pont entre le groupe A et le groupe Z permet au sein du ligand un transfert d'électrons photoinduit privilégié du groupe le plus donneur vers le groupe le plus accepteur, propriété très recherchée dans les domaines où se produisent des phénomènes de transfert d'électrons photoinduits.

Il est à noter également que, si le groupe triazole permet un transfert rapide d'électrons entre le groupe A et Z ou vice-versa, il n'altère pour autant en rien les états excités de l'élément métallique complexé par le groupe A.

Les composés de l'invention et les complexes de coordination en découlant, du fait de la capacité à transférer rapidement des électrons du groupe A vers le groupe Z et vice-versa, qui lui-même peut rentrer en contact avec une autre entité qui va accepter ledit électron (par exemple, une électrode, un composé chimique, une cellule biologique) vont trouver une application dans tous les domaines où le transfert d'électrons est nécessaire, ce qui est le cas dans les domaines suivants :
- le domaine de production de carburants impliquant des réactions d'oxydo-réduction, tel que la production d'hydrogène et d'oxygène par décomposition photoinduite de l'eau ou la réduction photoinduite du dioxyde de carbone, ces réactions constituant des moyens écocompatibles de produire des carburants à partir d'une source d'énergie renouvelable, l'énergie lumineuse ; dans ce cas, les complexes de l'invention peuvent être inclus dans une cellule de photocatalyse, plus particulièrement dans une photoanode, par exemple en dioxyde de titane, où se produit l'oxydation du réactif (par exemple, l'eau s'oxydant en oxygène), les électrons produits par cette réaction étant conduits ensuite vers une photocathode où se produit la réduction d'un autre réactif (par exemple, la réduction de protons), les produits issus des réactions d'oxydation et de réduction pouvant être utilisés comme carburant ;
- le domaine de production de produits de synthèse, lesquels sont fabriqués par oxydation ou réduction d'un précurseur ;
- le domaine thérapeutique ou du diagnostic, dans lequel il peut être nécessaire d'oxyder ou de réduire une molécule biologique ;
- le domaine de la production de l'électricité, les complexes de l'invention pouvant ainsi entrer dans la constitution de dispositifs photovoltaïques.

Il s'entend que d'autres applications que celles susmentionnées peuvent être envisagées, dès lors qu'elles impliquent un transfert d'électrons photoinduit.

L'invention va être à présent décrite en rapport aux exemples suivants donnés à titre illustratif et non limitatif.

### EXPOSÉ DÉTAILLÉ DE MODES DE RÉALISATION PARTICULIERS

### EXEMPLE 1

Cet exemple illustre la préparation d'un composé précurseur nécessaire à la préparation des composés de l'invention : le 4'-azido-2,2'-bipyridine-N'-oxyde, ce composé intermédiaire répondant à la formule suivante :

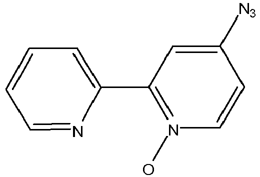

Ce composé précurseur est préparé par nitration du 2,2'-bipyridine-N'-oxyde pour donner du 4'-nitro-2,2'-bipyridine-N'-oxyde suivi d'un déplacement du groupe nitro par un groupe azoture, ces réactions pouvant être résumées par le schéma réactionnel suivant :

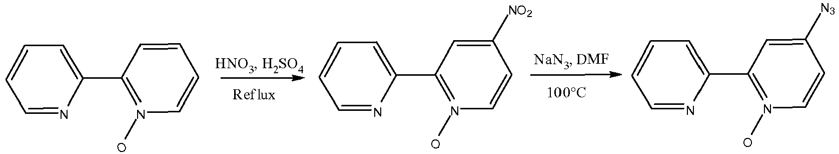

Le protocole opératoire de préparation du 4'-nitro-2,2'-bipyridine-N'-oxyde est le suivant :
De la 2,2'-bipyridine-N'-oxyde (2,00 g, 11,6 mmol) est dissoute dans de l'acide sulfurique concentré (12,0 mL, 22,1 g, 225 mmol, 19,4 éq) sous agitation. De l'acide nitrique fumant (19,0 mL, 28,9 g, 459 mmol, 39,5 éq) dans de l'acide sulfurique concentré (10,0 mL, 18,4 g, 188 mmol, 16,2 éq) est ajouté pendant 10 minutes dans le mélange précédent et le mélange réactionnel ainsi obtenu est porté à reflux (à savoir, à 120°C) pendant 4,5 heures. Le mélange réactionnel est ensuite versé dans de la glace (80 g) et neutralisé, avec refroidissement au bain de glace, par addition d'un solution aqueuse de soude à 38% jusqu'à obtention d'un pH de 8. Le précipité formé de couleur légèrement jaune est filtré et lavé à l'eau. Le précipité solide est ensuite dissous dans du chlorure de méthylène, de l'eau est ajoutée et le mélange résultant est extrait avec du chlorure de méthylène. Les phases organiques sont ensuite combinées, séchées avec du sulfate de sodium, filtrées puis concentrées pour donner 834 mg (33% de rendement) d'un solide beige consistant en du 4'-nitro-2,2'-bipyridine-N'-oxyde de formule suivante :

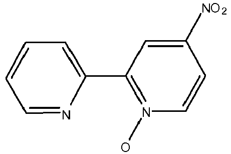

Le protocole opératoire de préparation du 4'-azido-2,2'-bipyridine-N'-oxyde est le suivant :
Du 4'-nitro-2,2'-bipyridine-N'-oxyde (400 mg, 1,84 mmol, 1 éq.) et de l'azoture de sodium (426 mg, 6,55 mmol, 3, 6 éq.) ont été mis en suspension dans du diméthylformamide (DMF) anhydre (20,0 mL) puis chauffés à 100°C pendant 20 heures sous une atmosphère d'argon. Après évaporation, de l'eau est ajoutée (25 mL) et le mélange est extrait avec du chlorure de méthylène (3*20 mL). Les phases organiques combinées sont séchées avec du sulfate de sodium, filtrées et concentrées pour donne une huile brunâtre. Le matériau brut obtenu est purifié par chromatographie sur colonne avec gel de silice (CH₂Cl₂/CH₃OH, 95/5) pour donner 190 mg (soit 49% de rendement) de 4'-azido-2,2'-bipyridine-N'-oxyde de formule suivante :

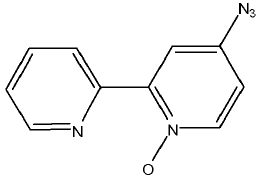

### EXEMPLE 2

Cet exemple illustre la préparation d'un composé conforme à l'invention : le 4'-(4"-phényl-1H-1", 2",3"-triazol-1"-yl)-2,2'-bipyridine répondant à la formule suivante :

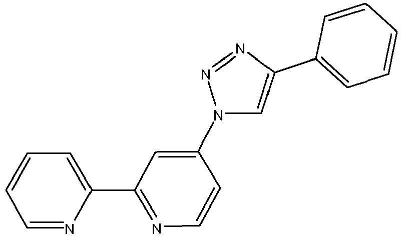

Ce composé est préparé en deux étapes : une première étape de préparation du 4'-(4"-phényl-1H-1", 2",3"-triazol-1"-yl)-2,2'-bipyridine-N'-oxyde et une seconde étape de préparation du 4'-(4"-phényl-1H-1", 2", 3"-triazol-1"-yl)-2,2'-bipyridine, ces étapes pouvant être résumées par le schéma réactionnel suivant :

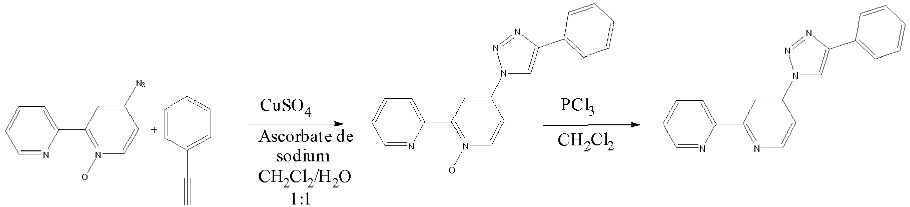

### a) Préparation du 4'-(4"-phényl-1H-1",2",3"-triazol-1"-yl)-2,2'-bipyridine-N'-oxyde

De la 4'-azido-2,2'-bipyridine-N'-oxyde préparée selon l'exemple 1 (42,6 mg, 200 µmol, 1 éq.) est mise en suspension dans du chlorure de méthylène (1,67 mL) sous une atmosphère d'argon. Du phénylacétylène (22,0 µL, 20,4 mg, 200 µmol, 1 éq.) est ajouté au mélange précédent, puis de l'eau (1,51 mL), de l'ascorbate de sodium (80 µL, 49,5 mg par mL d'eau, 20 µmol, 0,1 éq.) et du pentahydrate de sulfate de cuivre (80 µL, 31,2 mg par mL d'eau, 10 µmol, 0,05 éq.) sont ajoutés de manière séquentielle. Le mélange réactionnel est agité à température ambiante pendant 20 heures. Après cette durée, une chromatographie sur couche mince dans CH₂Cl₂/Méthanol (9/1) montre que la réaction de couplage est quantitative. Le mélange réactionnel est ensuite dilué avec 6 mL d'un mélange CH₂Cl₂/H₂O (1/1) et est extrait avec du chlorure de méthylène (3*6mL). Les phases organiques sont combinées et lavées avec de l'eau, séchées avec du sulfate de sodium, filtrées et concentrées pour donner 61,5 mg (soit 98% de rendement) d'un solide légèrement jaune consistant en du 4'-(4"-phényl-1H-1",2",3"-triazol-1"-yl)-2,2'-bipyridine-N'-oxyde de formule suivante :

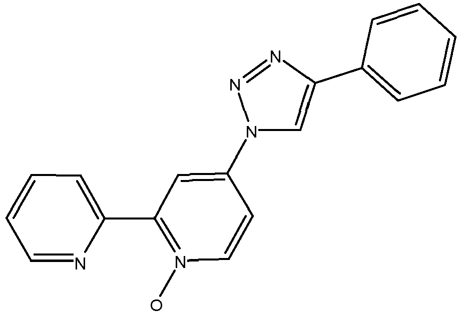

### b) Préparation du 4'-(4"-phényl-1H-1", 2", 3"-triazol-1"-yl)-2,2'-bipyridine

A une solution de 4'-(4"-phényl-1H-1", 2", 3"-triazol-1"-yl)-2,2'-bipyridine-N'-oxyde préparée préalablement (59,8 mg, 190 µmol, 1 éq.) dans du chlorure de méthylène (3,8 mL) sous une atmosphère d'argon, est ajouté du trichlorure de phosphore (49,7 µL, 78,3 mg, 570 µmol, 3,0 éq.) à 0°C. Le mélange réactionnel est porté à reflux pendant 3 heures, puis versé dans 4 mL de glace et neutralisé avec une solution aqueuse d'hydroxyde de sodium à 38,5%. La phase aqueuse est extraite plusieurs fois avec du chlorure de méthylène (3*5 mL). Les phases organiques sont ensuite combinées et lavées avec de l'eau, puis séchées avec du sulfate de sodium, filtrées et concentrées. Le résidu est purifié par chromatographie flash sur gel de silice (CH₂Cl₂/Methanol, 95/5), pour donner 49,2 mg (soit 87 % de rendement) d'un solide légèrement jaune correspondant à la 4'-(4"-phényl-1H-1", 2", 3"-triazol-1"-yl)-2,2'-bipyridine de formule suivante :

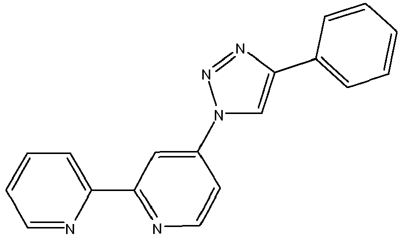

Un complexe de coordination de formule suivante : 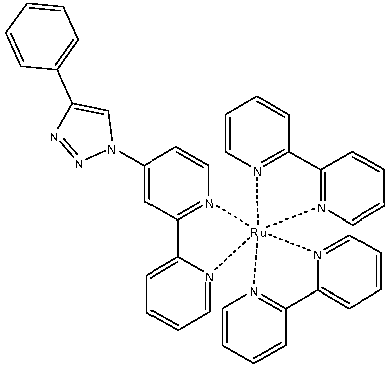 a été préparé à partir du composé synthétisé ci-dessus et de composés bipyridines.

Il a été procédé aux mesures des spectres d'absorption et d'émission de ce complexe, lesquels présentent un pic d'absorption maximale à 458 nm et un pic d'émission maximale à 632 nm (par rapport, respectivement à 451 nm et 608 nm pour un complexe de ruthénium comprenant trois groupes bipyridines non substitués). L'on peut ainsi en déduire que la présence d'un groupe triazole ne perturbe pas les caractéristiques d'absorption et d'émission du chromophore à base de ruthénium.

### EXEMPLE 3

Cet exemple illustre la préparation d'un composé conforme à l'invention : le 4'-(4"-(4"'-(diméthylamino)phényl)-1H-1", 2", 3"-triazol-1"-yl)-2,2'-bipyridine répondant à la formule suivante :

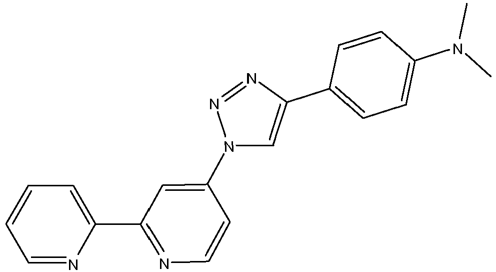

Ce composé est préparé en deux étapes : une première étape de préparation du 4'-(4"-(4"'-(diméthylamino)phényl)-1H-1",2",3"-triazol-1"-yl)-2,2'-bipyridine-N'-oxyde et une seconde étape de préparation du 4'-(4"-(4"'-(diméthylamino)phényl)-1H-1", 2", 3"-triazol-1"-yl)-2,2'-bipyridine, ces étapes pouvant être résumées par le schéma réactionnel suivant :

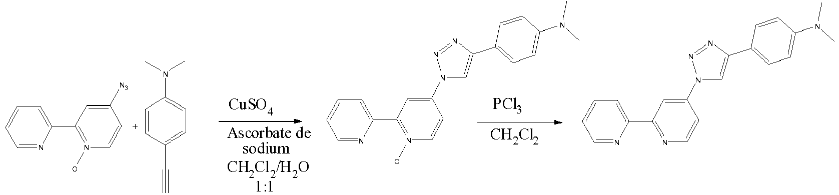

### a) Préparation du 4'-(4"-(4"'-(diméthylamino)phényl)-1H-1",2",3"-triazol-1"-yl)-2,2'-bipyridine-N'-oxyde

De la 4'-azido-2,2'-bipyridine-N'-oxyde préparée selon l'exemple 1 (32,0 mg, 150 µmol, 1 éq.) et du 4-éthynyl-N,N-diméthylaniniline (22,5 mg, 97%, 150 µmol, 1 éq.) sont mis en suspension dans du chlorure de méthylène (1,20 mL) sous une atmosphère d'argon. De l'eau (1,08 mL) est ajoutée au mélange réactionnel suivi par de l'ascorbate de sodium (60 µL, 49,5 mg par mL d'eau, 15 µmol, 0,1 éq.) et du pentahydrate de sulfate de cuivre (60 µL, 31,2 mg par mL d'eau, 7,5 µmol, 0,05 éq.) sont ajoutés de manière séquentielle. Le mélange réactionnel est agité à température ambiante pendant 20 heures. Après cette durée, une chromatographie sur couche mince dans CH₂Cl₂/Méthanol (9/1) montre que la réaction de couplage est quantitative. Le mélange réactionnel est ensuite dilué avec 5 mL d'un mélange CH₂Cl₂/H₂O (1/1) et est extrait avec du chlorure de méthylène (3*5 mL). Les phases organiques sont combinées et lavées avec l'eau, séchées avec du sulfate de sodium, filtrées et concentrées pour donner 51,4 mg (soit 96% de rendement) d'un solide orange correspondant à du 4'-(4"-(4"'-(diméthylamino)-phényl)-1H-1",2",3"-triazol-1"-yl)-2,2'-bipyridine-N'-oxyde de formule suivante :

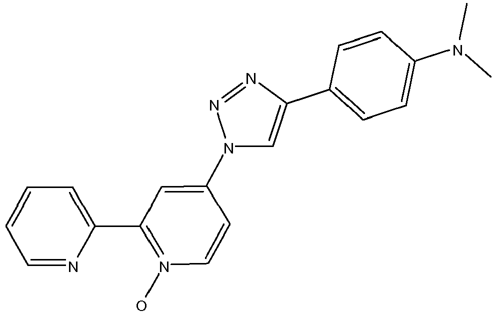

### b) Préparation du 4'-(4"-(4"'-(diméthylamino)phényl)-1H-1",2",3"-triazol-1"-yl)-2,2'-bipyridine

A une solution de 4'-(4"-(4"'-(diméthylamino)phényl)-1H-1",2",3"-triazol-1"-yl)-2,2'-bipyridine-N'-oxyde préparée préalablement (53,4 mg, 149 µmol, 1 éq.) dans du chlorure de méthylène (3,0 mL) sous une atmosphère d'argon, est ajouté du trichlorure de phosphore (39,0 µL, 61,4 mg, 447 µmol, 3,0 éq.) à 0°C. Le mélange réactionnel est porté à reflux pendant 3 heures, puis versé dans 3 mL de glace et neutralisé avec une solution aqueuse d'hydroxyde de sodium à 38,5%. La phase aqueuse est extraite plusieurs fois avec du chlorure de méthylène (3*5 mL). Les phases organiques sont ensuite combinées et lavées avec de l'eau, puis séchées avec du sulfate de sodium, filtrées et concentrées. Le résidu est purifié par chromatographie flash sur gel de silice (CH₂Cl₂/Methanol, 95/5), pour donner 40,1 mg (soit 79 % de rendement) d'une poudre jaune correspondant à la 4'-(4"-(4"'-(diméthylamino)phényl-1H-1",2",3"-triazol-1"-yl)-2,2'-bipyridine de formule suivante :

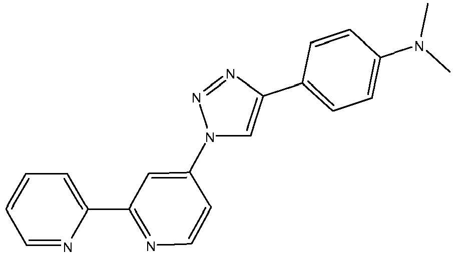

Un complexe de coordination de formule suivante : 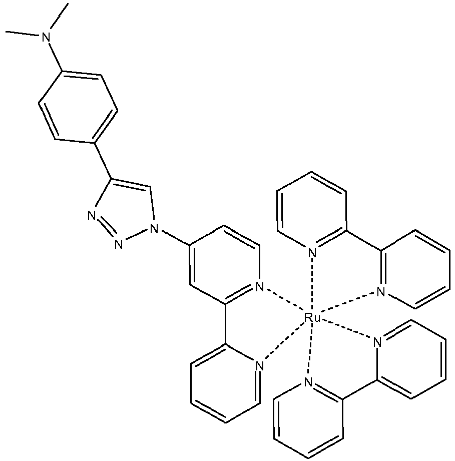 a été préparé à partir du composé synthétisé ci-dessus et de composés bipyridines.

Il a été procédé aux mesures des spectres d'absorption et d'émission de ce complexe, lesquels présentent un pic d'absorption maximale à 458 nm et un pic d'émission maximale à 627 nm (par rapport, respectivement à 451 nm et 608 nm pour un complexe de ruthénium comprenant trois groupes bipyridines non substitués). L'on peut ainsi en déduire que la présence d'un groupe triazole ne perturbe pas les caractéristiques d'absorption et d'émission du chromophore à base de ruthénium.

Des essais effectués avec ce complexe en présence de méthylviogène (accepteur d'électrons externe) (10 mM de méthylviogène dans l'acétonitrile) sous une lumière d'excitation (Energie : 10 mJ ; longueur d'onde λ=450 nm ; Absorbance A=0,36) ont permis de confirmer un transfert intramoléculaire d'électrons du groupe diméthylamino vers le chromophore comprenant le ruthénium (sous forme Ru³⁺). Ce transfert est très rapide (inférieur à 50 ns) comme indiqué par la cinétique de récupération de l'état Ru²⁺. L'état réduit de l'accepteur d'électrons externe est formé ainsi très rapidement et s'avère être stable pour des centaines de microsecondes.

Ainsi, l'on peut en déduire que le transfert d'électrons du groupe diméthylamino vers le chromophore comprenant le ruthénium via le groupe triazole est très efficace.

A titre comparatif, des essais similaires ont été réalisé avec un complexe de formule suivante :

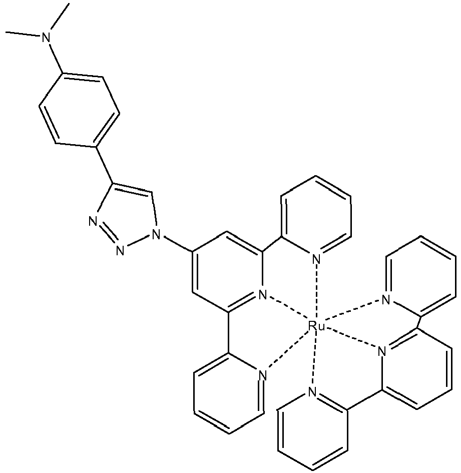

Il a été constaté que le transfert intramoléculaire d'électrons entre le groupe diméthylamino et le chromophore comprenant du ruthénium à travers le groupe triazole est beaucoup moins efficace (temps de transfert supérieur à 20 µs) que pour le complexe de l'invention (temps de transfert inférieur à 50 ns).

### EXEMPLE 4

Cet exemple illustre la préparation d'un composé conforme à l'invention : le 4'-(4",(4"'-fluorophényl)-1H-1",2",3"-triazol-1"-yl)-2,2'-bipyridine répondant à la formule suivante :

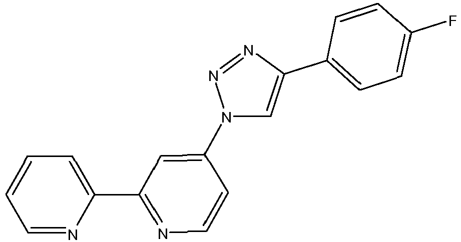

Ce composé est préparé en deux étapes : une première étape de préparation du 4'-(4"-(4"'-fluorophényl)-1H-1",2",3"-triazol-1"-yl)-2,2'-bipyridine-N'-oxyde et une seconde étape de préparation du 4'-(4"-(4"'-fluorophényl)-1H-1",2",3"-triazol-1"-yl)-2,2'-bipyridine, ces étapes pouvant être résumées par le schéma réactionnel suivant :

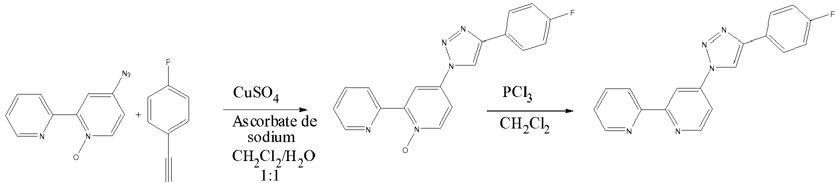

### a) Préparation du 4'-(4"-(4"'-fluorophényl)-1H-1",2",3"-triazol-1"-yl)-2,2'-bipyridine-N'-oxyde

De la 4'-azido-2,2'-bipyridine-N'-oxyde préparée selon l'exemple 1 (32,0 mg, 150 µmol, 1 éq.) est mise en suspension dans du chlorure de méthylène (1,20 mL) sous une atmosphère d'argon. Du 1-éthynyl-4-fluorobenzène (17,5 µL, 18,4 mg, 98%, 150 µmol, 1 éq.) est ajouté à la suspension, suivi par de l'eau (1,08 mL), de l'ascorbate de sodium (60 µL, 49,5 mg par mL d'eau, 15 µmol, 0,1 éq.) et du pentahydrate de sulfate de cuivre (60 µL, 31,2 mg par mL d'eau, 7,5 µmol, 0,05 éq.) sont ajoutés de manière séquentielle. Le mélange réactionnel est agité à température ambiante pendant 20 heures. Après cette durée, une chromatographie sur couche mince dans CH₂Cl₂/Méthanol (9/1) montre que la réaction de couplage est quantitative. Le mélange réactionnel est ensuite dilué avec 5 mL d'un mélange CH₂Cl₂/H₂O (1/1) et est extrait avec du chlorure de méthylène (3*5 mL). Les phases organiques sont combinées et lavées avec l'eau, séchées avec du sulfate de sodium, filtrées et concentrées pour donner 49,2 mg (soit 98% de rendement) d'un solide jaune correspondant à du 4'-(4"-(4"'-fluorophényl)-1H-1",2", 3"-triazol-1"-yl)-2,2'-bipyridine-N'-oxyde de formule suivante :

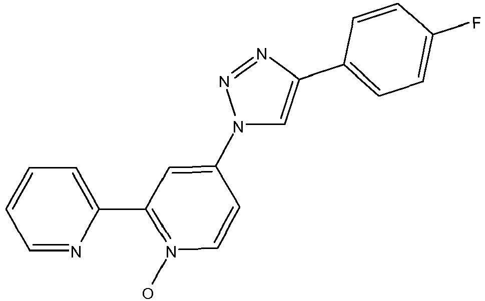

### b) Préparation du 4'-(4"-(4"'-fluorophényl)-1H-1",2",3"-triazol-1"-yl)-2,2'-bipyridine

A une solution de 4'-(4"-(4"'-fluorophényl)-1H-1",2",3"-triazol-1"-yl)-2,2'-bipyridine-N'-oxyde préparée préalablement (47,5 mg, 143 µmol, 1 éq.) dans du chlorure de méthylène (2,9 mL) sous une atmosphère d'argon, est ajouté du trichlorure de phosphore (37,4 µL, 58,9 mg, 429 µmol, 3,0 éq.) à 0°C. Le mélange réactionnel est porté à reflux pendant 3 heures, puis versé dans 3 mL de glace et neutralisé avec une solution aqueuse d'hydroxyde de sodium à 38,5%. La phase aqueuse est extraite plusieurs fois avec du chlorure de méthylène (3*5 mL). Les phases organiques sont ensuite combinées et lavées avec de l'eau, puis séchées avec du sulfate de sodium, filtrées et concentrées. Le résidu est purifié par chromatographie flash sur gel de silice (CH₂Cl₂/Méthanol, 95/5), pour donner 37,2 mg (soit 82 % de rendement) d'une poudre légèrement jaune correspondant à la 4'-(4"-(4"'-fluorophényl-1H-1",2",3"-triazol-1"-yl)-2,2'-bipyridine de formule suivante :

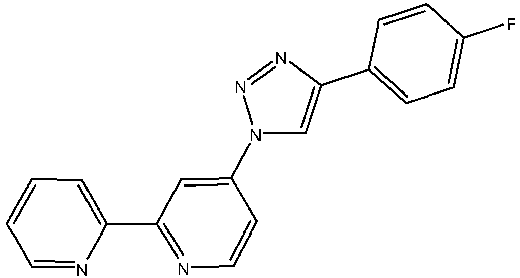

Un complexe de coordination de formule suivante : 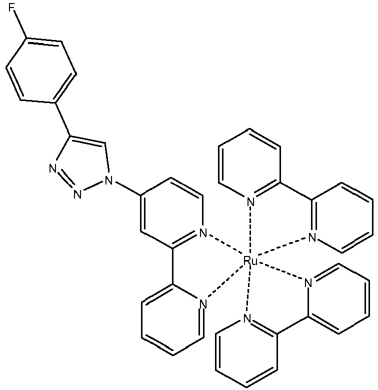 a été préparé à partir du composé synthétisé ci-dessus et de composés bipyridines.

Il a été procédé aux mesures des spectres d'absorption et d'émission de ce complexe, lesquels présentent un pic d'absorption maximale à 458 nm et un pic d'émission maximale à 633 nm (par rapport, respectivement à 451 nm et 608 nm pour un complexe de ruthénium comprenant trois groupes bipyridines non substitués). L'on peut ainsi en déduire que la présence d'un groupe triazole ne perturbe pas les caractéristiques d'absorption et d'émission du chromophore à base de ruthénium.

### EXEMPLE 5

Cet exemple illustre la préparation d'un composé conforme à l'invention : le 4'-(4", (4"'-(méthoxycarbonyl)phényl)-1H-1",2",3"-triazol-1"-yl)-2,2'-bipyridine répondant à la formule suivante :

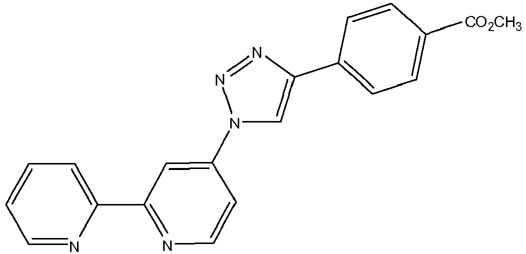

Ce composé est préparé en deux étapes : une première étape de préparation du 4'-(4"-(4"'-(méthoxycarbonyl)phényl)-1H-1",2",3"-triazol-1"-yl)-2,2'-bipyridine-N'-oxyde et une seconde étape de préparation du 4'-(4"-(4"'-(méthoxycarbonyl)phényl)-1H-1",2",3"-triazol-1"-yl)-2,2'-bipyridine, ces étapes pouvant être résumées par le schéma réactionnel suivant :

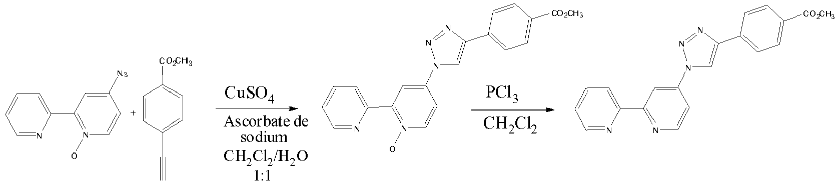

### a) Préparation du 4'-(4"-(4"'-(méthoxycarbonyl)phényl)-1H-1",2",3"-triazol-1"-yl)-2,2'-bipyridine-N'-oxyde

De la 4'-azido-2,2'-bipyridine-N'-oxyde préparée selon l'exemple 1 (32,0 mg, 150 µmol, 1 éq.) est mise en suspension dans du chlorure de méthylène (2,50 mL) sous une atmosphère d'argon. Du méthyl-4-éthynylbenzoate (24,0 mg, 150 µmol, 1 éq.) est ajouté à la suspension, suivi par de l'eau (2,38 mL), de l'ascorbate de sodium (60 µL, 49,5 mg par mL d'eau, 15 µmol, 0,1 éq.) et du pentahydrate de sulfate de cuivre (60 µL, 31,2 mg par mL d'eau, 7,5 µmol, 0,05 éq.) sont ajoutés de manière séquentielle. Le mélange réactionnel est agité à température ambiante pendant 40 heures. Après cette durée, une chromatographie sur couche mince dans CH₂Cl₂/Méthanol (9/1) montre que la réaction de couplage est quantitative. Le mélange réactionnel est ensuite dilué avec 10 mL d'un mélange CH₂Cl₂/H₂O (1/1) et est extrait avec du chlorure de méthylène (3*10 mL). Les phases organiques sont combinées et lavées avec l'eau, séchées avec du sulfate de sodium, filtrées et concentrées pour donner 54,8 mg (soit 98% de rendement) d'un solide jaune correspondant à du 4'-(4"-(4"'-(méthoxycarbonyl)phényl)-1H-1",2",3"-triazol-1"-yl)-2,2'-bipyridine-N'-oxyde de formule suivante :

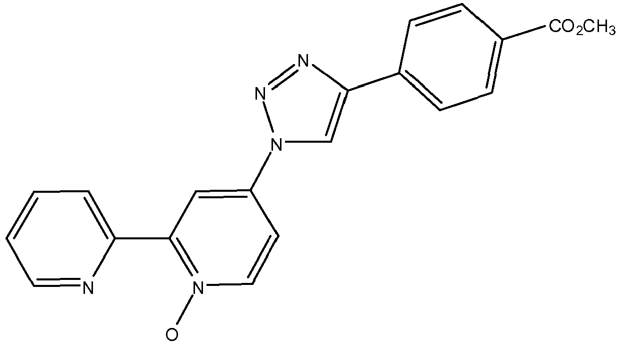

### b) Préparation du 4'-(4"-(4"'-(méthoxycarbonyl)phényl)-1H-1",2",3"-triazol-1"-yl)-2,2'-bipyridine

A une solution de 4'-(4"-(4"'-(méthoxycarbonyl)phényl)-1H-1",2",3"-triazol-1"-yl)-2,2'-bipyridine-N'-oxyde préparée préalablement (54,8 mg, 147 µmol, 1 éq.) dans du chlorure de méthylène (6,0 mL) sous une atmosphère d'argon, est ajouté du trichlorure de phosphore (80,0 µL, 126,0 mg, 916 µmol, 6,2 éq.) à 0°C. Le mélange réactionnel est porté à reflux pendant 3 heures, puis versé dans 5 mL de glace et neutralisé avec une solution aqueuse d'hydroxyde de sodium à 38,5%. La phase aqueuse est extraite plusieurs fois avec du chlorure de méthylène (3*5 mL). Les phases organiques sont ensuite combinées et lavées avec de l'eau, puis séchées avec du sulfate de sodium, filtrées et concentrées. Le résidu est purifié par chromatographie flash sur gel de silice (CH₂Cl₂/Méthanol, 97/3), pour donner 34,0 mg (soit 65 % de rendement) d'une poudre légèrement jaune correspondant à la 4'-(4"-(4"'-(méthoxycarbonyl)phényl-1H-1",2",3"-triazol-1"-yl)-2,2'-bipyridine de formule suivante :

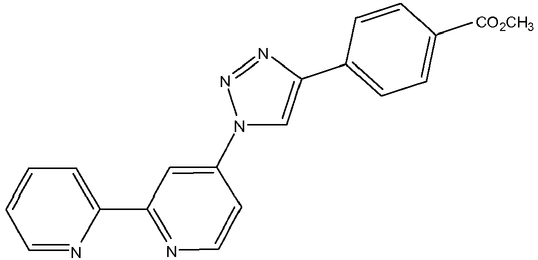

Un complexe de coordination de formule suivante : 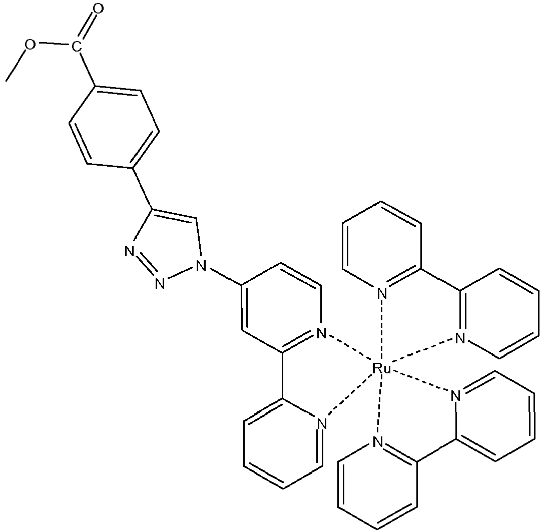 a été préparé à partir du composé synthétisé ci-dessus et de composés bipyridines.

Ce complexe a été ensuite ancré sur un substrat en dioxyde de titane TiO₂ via le groupe ester -CO₂CH₃. Le spectre d'émission du complexe ainsi ancré a été élaboré et montre un pic d'émission maximale vers 640 nm.

L'on peut en déduire ainsi que le groupe triazole permet un transfert d'électrons du chromophore comprenant du ruthénium vers le substrat.

## Revendications

1. Composé répondant à la formule (I) suivante :
A-T-Z (I)
dans laquelle :
*A est un groupe apte à complexer au moins un élément métallique, qui est un groupe bipyridine,
le groupe bipyridine répondant à la formule suivante : 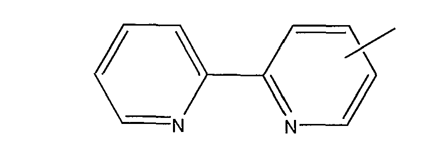
la liaison située au milieu de la liaison carbone-carbone indiquant que l'attachement au groupe triazole peut se faire par l'un quelconque des atomes de carbone constitutif du cycle bipyridine ;
*T est un groupe triazole directement lié au groupe A, ledit groupe T répondant à la formule suivante : 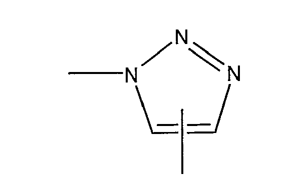
la liaison coupant la double liaison carbone-carbone indiquant que le groupe triazole est lié au groupe A ou groupe Z via l'un des atomes de carbone constitutif de cette double liaison tandis que la liaison attachée à l'atome d'azote indique que le triazole est lié à l'autre groupe (A ou Z) par le biais de cet atome d'azote ;
*Z est un atome d'halogène, un groupe nitro, un groupe cyano, un groupe alkyle, un groupe aryle, un groupe hétérocyclique, un groupe de formule -NR¹R², un groupe de formule -SR¹, un groupe de formule -S(=O)₂(OR¹), un groupe de formule -O-S(=O)₂(OR¹), un groupe de formule -O-S(=O)₂(R¹), un groupe de formule -S(=O)(OR¹), un groupe de formule -S(=O)(R¹) , un groupe de formule -S(=O)₂R¹, un groupe de formule -PR¹R², un groupe de formule -P(=O)(OR¹)(OR²), un groupe de formule -O-P(=O)(OR¹)(OR²), un groupe de formule -O-P(=O)(OR¹)(R²), un groupe de formule -OR¹ ou un groupe de formule -CO-R¹, R¹ et R² représentant indépendamment un atome d'hydrogène, un groupe alkyle ou un groupe aryle, lesdits groupes alkyles ou aryles étant éventuellement substitués.

2. Composé selon la revendication 1, dans lequel le groupe Z est un groupe aryle éventuellement substitué.

3. Composé selon la revendication 2, dans lequel le groupe aryle est substitué par au moins un groupe -NR¹R², au moins un groupe -CO₂R¹, R¹ et R² étant tels que définis à la revendication 1 et/ou au moins un atome d'halogène.

4. Composé selon l'une quelconque des revendications 1 à 3, répondant à la formule (II) suivante : 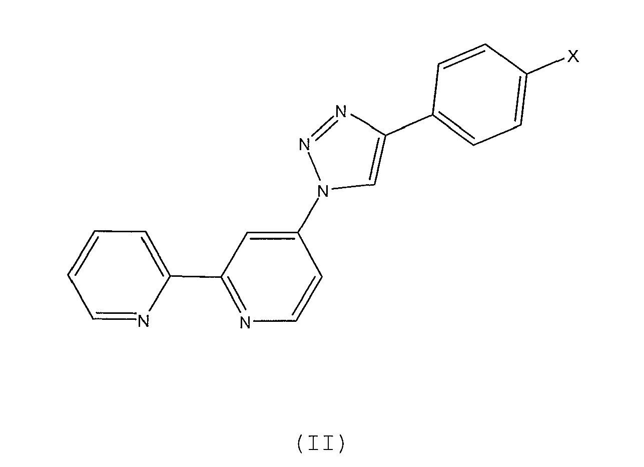 X représentant un atome d'hydrogène, un atome d' halogène, un groupe -NR¹R² ou un groupe -CO₂R¹, R¹ et R² répondant à la même définition que celle donnée à la revendication 1.

5. Composé selon la revendication 4, dans lequel X est un atome d'hydrogène.

6. Composé selon la revendication 4, dans lequel X est un groupe -N(CH₃)₂.

7. Composé selon la revendication 4, dans lequel X est un composé de formule -CO₂CH₃.

8. Composé selon la revendication 4, dans lequel X est un atome de fluor.

9. Complexe de coordination d'au moins un élément métallique avec au moins un composé tel que défini selon l'une quelconque des revendications 1 à 8.

10. Complexe de coordination selon la revendication 9, dans lequel l'élément métallique est choisi parmi les métaux de transition, les éléments lanthanides, les éléments actinides, les éléments Al, Ga, Ge, In, Sn, Sb, Tl, Pb, Bi et Po.

11. Complexe de coordination selon la revendication 9 ou 10, dans lequel l'élément métallique est un métal de transition.

12. Complexe de coordination selon l'une quelconque des revendications 9 à 11, dans lequel l'élément métallique est du ruthénium.

13. Complexe de coordination selon l'une quelconque des revendications 9 à 12, comprenant en outre un composé ligand choisi parmi la bipyridine, la pyridine.

14. Complexe de coordination selon l'une quelconque des revendications 9 à 13 répondant à la formule (III) suivante : 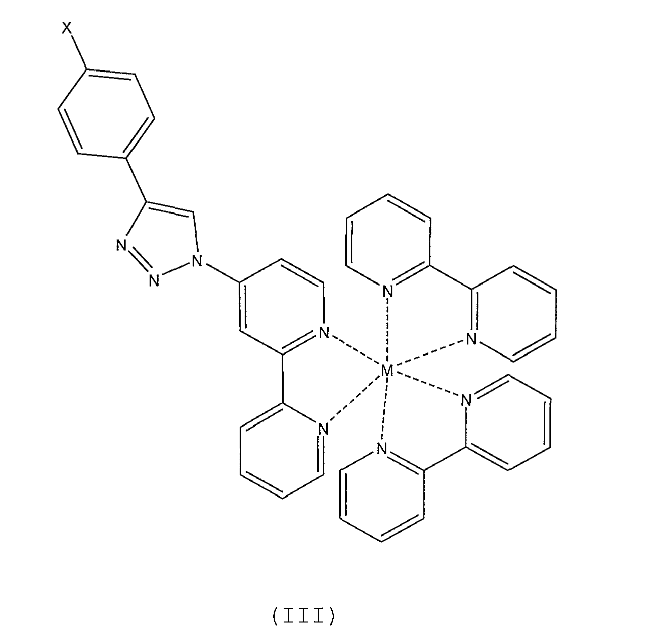 avec X étant tel que défini à la revendication 4 et M étant un élément métallique.

## Patentansprüche

1. Verbindung, die der nachfolgenden Formel (I) entspricht:
A-T-Z (I)
worin:
*A eine Gruppe ist, die dazu geeignet ist, zumindest ein metallisches Element zu komplexieren und die eine Bipyridin-Gruppe ist,
wobei die Bipyridin-Gruppe der nachfolgenden Formel entspricht: 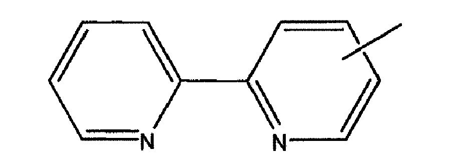
wobei die in der Mitte der Kohlenstoff-Kohlenstoff-Bindung liegende Bindung angibt, dass die Anbindung an die Triazol-Gruppe über ein beliebiges der Kohlenstoffatome erfolgen kann, das Bestandteil des Bipyridin-Rings ist;
*T eine Triazol-Gruppe ist, die direkt mit der Gruppe A verbunden ist, wobei die Gruppe T der nachfolgenden Formel entspricht: 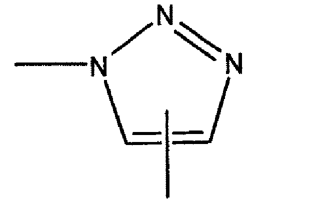
wobei die die Kohlenstofif-Kohlenstofif-Doppelbindung schneidende Bindung angibt, dass die Triazol-Gruppe mit der Gruppe A oder der Gruppe Z über eines der Kohlenstoffatome verbunden ist, das Bestandteil dieser Doppelbindung ist, während die an das Stickstofifatom gebundene Bindung angibt, dass das Triazol mit der weiteren Gruppe (A bzw. Z) über dieses Stickstofifatom verbunden ist;
*Z ein Halogenatom, eine Nitro-Gruppe, eine Cyano-Gruppe, eine Alkyl-Gruppe, eine Aryl-Gruppe, eine heterocyclische Gruppe, eine Gruppe der Formel -NR¹R², eine Gruppe der Formel -SR¹, eine Gruppe der Formel -S(=O)₂(OR¹), eine Gruppe der Formel -O-S(=O)₂(OR¹), eine Gruppe der Formel -O-S(=O)₂(R¹), eine Gruppe der Formel -S(=O)(OR¹), eine Gruppe der Formel -S(=O)(R¹), eine Gruppe der Formel -S(=O)₂R¹, eine Gruppe der Formel -PR¹R², eine Gruppe der Formel -P(=O) (OR¹) (OR²), eine Gruppe der Formel -O-P (=O) (OR¹) (OR²), eine Gruppe der Formel -O-P (=O) (OR¹) (OR²), eine Gruppe der Formel -OR¹ oder eine Gruppe der Formel -CO-R¹, wobei R¹ und R² unabhängig ein Wasserstoff-Atom, eine Alkyl-Gruppe oder eine Aryl-Gruppe darstellen, wobei die Alkyl- bzw. Aryl-Gruppen gegebenenfalls substituiert sind.

2. Verbindung nach Anspruch 1, wobei die Gruppe Z eine gegebenenfalls substituierte Aryl-Gruppe ist.

3. Verbindung nach Anspruch 2, wobei die Aryl-Gruppe mit zumindest einer Gruppe -NR¹R², zumindest einer Gruppe -CO₂R¹ substituiert ist, wobei R¹ und R² wie in Anspruch 1 definiert und/oder zumindest ein Halogen-Atom sind.

4. Verbindung nach einem der Ansprüche 1 bis 3, wobei sie der nachfolgenden Formel (II) entspricht: 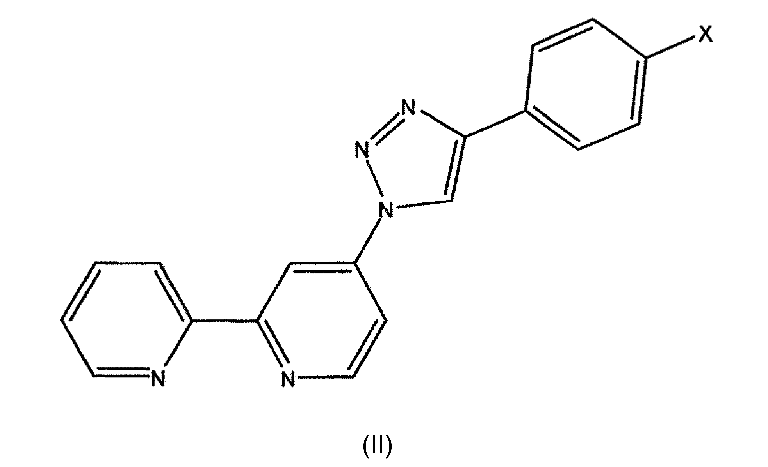 wobei X ein Wasserstoff-Atom, ein Halogen-Atom, eine -NR¹R² Gruppe oder eine -CO₂R¹ Gruppe darstellt, wobei R¹ und R² der gleichen Definition entsprechen, wie die in Anspruch 1 angegeben ist.

5. Verbindung nach Anspruch 4, wobei X ein Wasserstoff-Atom ist.

6. Verbindung nach Anspruch 4, wobei X eine -N(CH₃)₂ Gruppe ist.

7. Verbindung nach Anspruch 4, wobei X eine Verbindung der Formel -CO₂CH₃ ist.

8. Verbindung nach Anspruch 4, wobei X ein Fluor-Atom ist.

9. Koordinationskomplex von zumindest einem metallischen Element mit zumindest einer Verbindung wie nach einem der Ansprüche 1 bis 8 definiert.

10. Koordinationskomplex nach Anspruch 9, wobei das metallische Element ausgewählt ist aus Übergangsmetallen, Lanthanoiden, Actinoiden, den Elementen Al, Ga, Ge, In, Sn, Sb, TI, Pb, Bi und Po.

11. Koordinationskomplex nach Anspruch 9 oder 10, wobei das metallische Element ein Übergangsmetall ist.

12. Koordinationskomplex nach einem der Ansprüche 9 bis 11, wobei das metallische Element Ruthenium ist.

13. Koordinationskomplex nach einem der Ansprüche 9 bis 12, ferner enthaltend eine Ligand-Verbindung, die ausgewählt ist aus Bipyridin, Pyridin.

14. Koordinationskomplex nach einem der Ansprüche 9 bis 13, welcher der nachfolgenden Formel (III) entspricht: 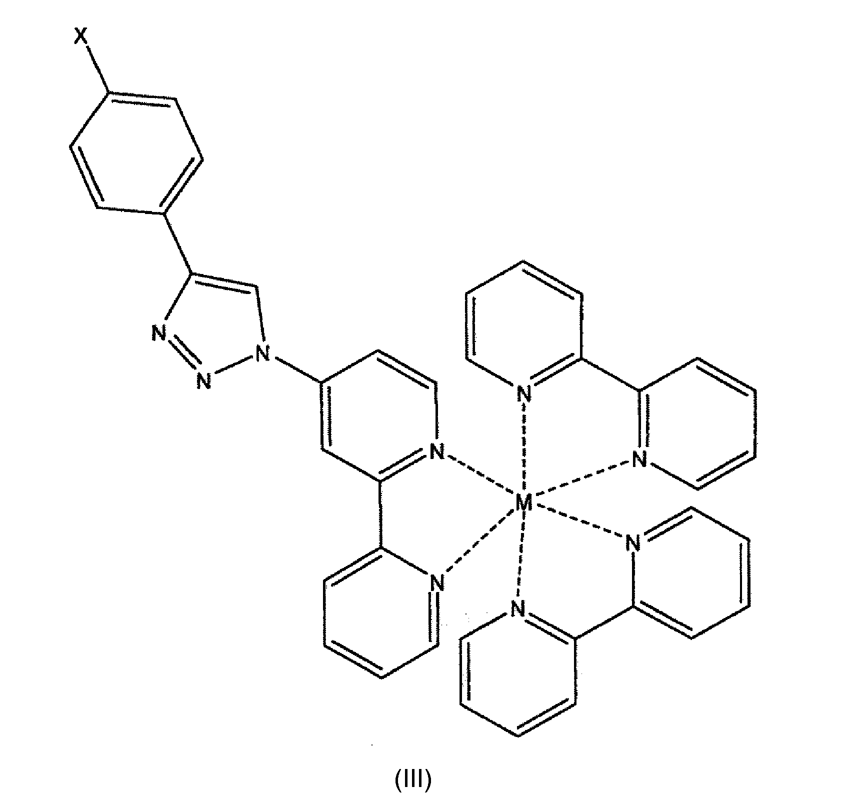 wobei X wie in Anspruch 4 definiert ist und M ein metallisches Element ist.

## Claims

1. A compound fitting the following formula (I) :
A-T-Z (I)
wherein:
* A is a group capable of complexing at least one metal element, which is a bipyridine, group,
said group bipyridine, having the following formula: 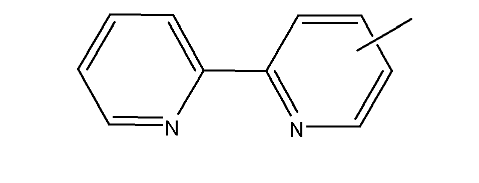
the bond located in the middle of the carbon-carbon bond indicating that the binding to the triazole group may be accomplished by any of the constitutive carbon atoms of the bipyridine ring;
* T is a triazole group directly bound to the group A, said triazole group fitting the following formula: 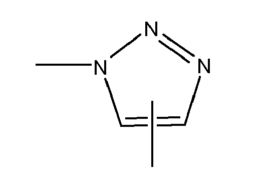 the bond cutting the carbon-carbon double bond indicating that the triazole group is bound to group A or group Z via one of the constitutive carbon atoms of this double bond while the bond attached to the nitrogen atom indicates that the triazole is bound to the other group (A or Z) by means of this nitrogen atom;
* Z is a halogen atom, a nitro group, a cyano group, an alkyl group, an aryl group, a heterocyclic group, a group of formula -NR¹R², a group of formula -SR¹, a group of formula -S(=O)₂(OR¹), a group of formula -O-S(=O)₂(OR¹), a group of formula -O-S (=O)₂(R¹), a group of formula -S(=O)(OR¹), a group of formula -S(=O)(R¹), a group of formula -S(=O)₂R¹, a group of formula -PR¹R², a group of formula -P(=O) (OR¹) (OR²) , a group of formula -O-P(=O) (OR¹) (OR²) , a group of formula -O-P(=O) (OR¹) (R²) , a group of formula -OR¹ or a group of formula -CO-R¹, R¹ and R² independently representing a hydrogen atom, an alkyl group or an aryl group, said alkyl or aryl groups being optionally substituted.

2. The compound according to claim 1, wherein the group Z is an optionally substituted aryl group.

3. The compound according to claim 2, wherein the aryl group is substituted with at least one group -NR¹R², at least one group -CO₂R¹, R¹ and R² being as defined in claim 1 and/or at least one halogen atom.

4. The compound according to any of claims 1 to 3, fitting the following formula (II): 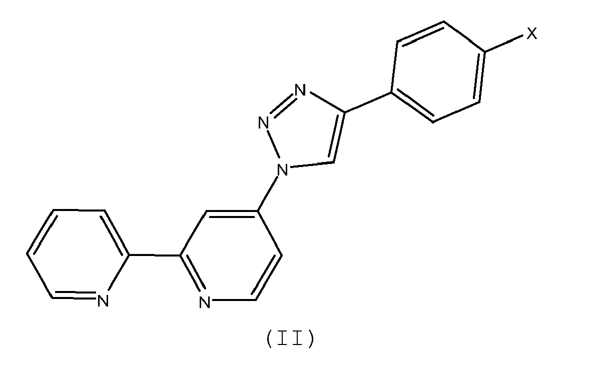 X representing a hydrogen atom, a halogen atom, a group -NR¹R² or a group -CO₂R¹, R¹ and R² fitting the same definition as the one given in claim 1.

5. The compound according to claim 4, wherein X is a hydrogen atom.

6. The compound according to claim 4, wherein X is a group -N(CH₃)₂.

7. The compound according to claim 4, wherein X is a compound of formula -CO₂CH₃.

8. The compound according to claim 4, wherein X is a fluorine atom.

9. A complex for coordination of at least one metal element with at least one compound as defined according to any of claims 1 to 8.

10. The coordination complex according to claim 9, wherein the metal element is selected from transition metals, lanthanide elements, actinide elements, elements Al, Ga, Ge, In, Sn, Sb, Tl, Pb, Bi and Po.

11. The coordination complex according to claim 9 or 10, wherein the metal element is a transition metal.

12. The coordination complex according to any of claims 9 to 11, wherein the metal element is ruthenium.

13. The coordination complex according to any of claims 9 to 12, further comprising a ligand compound selected from bipyridine, pyridine.

14. The coordination complex according to any of claims 9 to 13 fitting the following formula (III) : 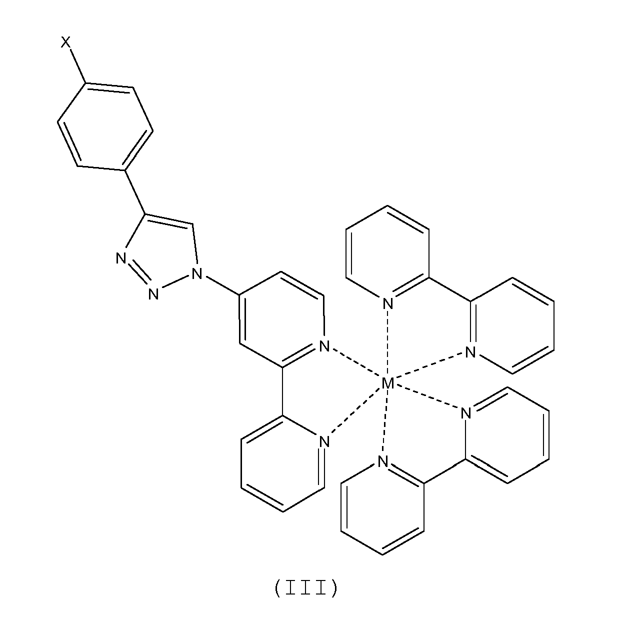 with X being as defined in claim 4 and M being a metal element.
